Europäisches Patentamt

European Patent Office (11) Publication number: **0 385 433**

Office européen des brevets **A2**

(12) **EUROPEAN PATENT APPLICATION**

(21) Application number: 90103896.8

(51) Int. Cl.⁵: **C07K 1/04, B01J 19/00**

(22) Date of filing: 28.02.90

(30) Priority: 28.02.89 CS 1280/89

(43) Date of publication of application:
05.09.90 Bulletin 90/36

(84) Designated Contracting States:
**DE FR GB IT SE**

(71) Applicant: **CESKOSLOVENSKA AKADEMIE VED**
Narodni 3
Praha 1(CS)

(72) Inventor: **Lébl, Michal**
**Rimská 36**
**Praha 2(CS)**
Inventor: **Gut, Vladimir**
**Vlkova 33**
**Praha 3(CS)**
Inventor: **Eichler, Jutta**
**Rückenstrasse 32**
**DDR-1055 Berlin(DD)**
Inventor: **Krchnák, Viktor**
**Talafúsova 6**
**Praha 4(CS)**
Inventor: **Vágner, Josef**
**Bitovská 1226**
**Praha 4(CS)**
Inventor: **Stepánek, Jiri**
**K Horomericum 5**
**Praha 6(CS)**

(74) Representative: **Patentanwälte Beetz sen. - Beetz jun. Timpe - Siegfried - Schmitt-Fumian- Mayr**
**Steinsdorfstrasse 10**
**D-8000 München 22(DE)**

(54) Method and apparatus for the continuous polymer synthesis on a solid carrier.

(57) The invention relates to a novel method and apparatus for the solid-phase synthesis of polymers such as polypeptides wherein an elongated, preferably band-like carrier (1) is used. The attachment of the reactants is realized by successive introduction of the carrier (1) into the appropriate baths. This method allows continuous preparation of polypeptides, polynucleotides, etc.
The apparatus comprises a solid, insoluble, elongated carrier (1) comprising functional groups and an arrangement of successive reaction zones (5, 7) and processing zones (4, 6, 8) of a sequence identical with that of the reaction and processing steps of the process sequence of the respective synthesis, and comprises means (2a, 2b) for successively contacting the carrier (1) with the reaction and processing liquids.

FIG. 1

# METHOD AND APPARATUS FOR THE CONTINUOUS POLYMER SYNTHESIS ON A SOLID CARRIER

This invention relates to a method, an apparatus and a carrier for the continuous synthesis of oligomer and macromolecular organic compounds and particularly of biopolymers such as oligo- and polysaccharides, oligo- and polynucleotides, and in particular oligo- and polypeptides on a solid carrier.

Peptides are prepared by classical methods in solution (Houben-Weyl, Methoden der Organischen Chemie, Synthese von Peptiden (E. Wünsch ed.) Thieme-Verlag, Berlin 1974) as well as by solid phase synthesis developed by Merrifield (for a recent review see e.g. Stewart, J.M., and Young, J.D., Solid Phase Peptide Synthesis, Freeman, San Francisco 1969; second edition 1985). The solid phase method has the advantage of a high spedd because during the synthesis the intermediates are isolated simply by collecting the solid carrier and washing it with solvents. This method has been shown to be suitable for automation (see e.g. Merrifield, R.B., Stewart, J.M., and Jernberg, N., Apparatus for the automated synthesis of peptides, US-A-3 531 258; Brunfeldt, K., Roepstorff, P., and Halstrom, J., Reaction system, US-A-3 557 077; Kubodera, T., Hara, T., and Makabe, H., Apparatus for synthesis of peptides or the like organic compounds, US-A-3 647 390; Won Kil Park and Regoli, D., System for the solid phase synthesis, US-A-3 715 190; Verlander, M.S., Fuller, W.D., and Goodman, M., Rapid, large scale, automatable high pressure peptide synthesis, US-A-4 192 798; Hamill, B.J., Apparatus for the chemical synthesis of oligonucleotides, US-A-4 728 502; Neimark, J., and Briand, J.-P., Semi-automatic, solid-phase peptide multi-synthesizer and process for the production of synthetic peptides by the use of multi-synthesizer, US-A-4 748 002; Bridgham, J., et al., Automated polypeptide synthesis apparatus, US-A-4 668 476; Saneii, H.H., Solid phase synthesizer, US-A-4 746 490).

Some other chemical and physico-chemical processes have been successfully modified for continuous operation (colouring of textiles (US-A-4 863 154), developing of films, etc.) and automated. Devices serving for similar purposes are used e.g. in glue spreading onto tapes (FR-A-2 627 108), cloth and paper impregnation (FR-A-2 612 086, US-A-4 863 085), belt driers (US-A-3 931 684), electrographical devices (US-A-3 783 827), or in the application of several liquid layers onto textiles (DE-Al-3 414 504).

Obviously, continuous performance of peptide synthesis would enable continuous production of these highly sophisticated organic compounds. Up to now, no attempts on the continuous modification of peptide synthesis have been made, mostly because no suitable continuous method and carriers have been available.

It is the object of this invention to provide a method, an apparatus, and carriers for the continuous synthesis of oligomeric and polymeric organic compounds and particularly of biopolymers, such as oligo- and polysaccharides, oligo- and polynucleotides, and in particular oligo- and polypeptides which are suitable for automation.

This object is achieved according to the claims. The dependent claims relate to preferred embodiments.

The method of the present invention for the continuous synthesis of oligomeric and polymeric organic compounds, particularly of biopolymers, and preferably of polypeptides and polynucleotides,
is characterized in that
- a solid, insoluble, elongated carrier is used comprising functional groups serving for anchoring the first reaction component of the process sequence of the respective synthesis either directly or by means of a spacer,
and
- the individual reaction and processing steps are carried out in successive zones of a sequence identical with that of the reaction and processing steps of the process sequence moving in one direction given by the longest dimension of the carrier.

In accordance with a preferred embodiment, all reaction and processing steps of the process sequence are carried out at the same time in different parts of the carrier.

In accordance with another preferred embodiment, the carrier is successively subjected to the reaction and processing steps of the process sequence.

It is preferred to move the carrier through the successive zones, preferably with a predetermined, constant speed.

Another preferred embodiment is characterized in that an open- or closed-loop carrier is used the length of which is at least such as to correspond to the total residence time necessary for all reaction and processing steps of the process sequence for a given moving speed.

For obtaining some or all intermediates and eventually also the desired final product, the synthesis process is stopped at a stage where these compounds corresponding to the respective series of reaction steps of the process sequence have been formed in the corresponding parts of the carrier, and these compounds are removed from the carrier either continuously or in a batch-like manner from the respective parts of the carrier.

The final product is preferably removed from the carrier after completion of the total process sequence in all parts of the carrier, preferably in a batch-like manner.

The process sequence comprises all reaction and processing (e.g. washing) steps in the necessary order which are required for the synthesis of a given polymer product.

In accordance with the general concept of this invention all the reactions needed for the synthesis of the given polymer proceed at the same time in different parts of the carrier. The carrier preferably is a planar insoluble material containing suitable functional groups for anchoring the reaction component of the process sequence, i.e. for example the first amino acid in the case of peptide synthesis and is successively brought into contact with the reagent used for the sequential solid-phase synthesis.

More specifically, the method according to this invention represents a modified form of the solid phase synthesis enabling continuous production.

The apparatus of this invention for the continuous synthesis of oligomeric and polymeric organic compounds, particularly for carrying out the above-defined method, comprises
- a solid, insoluble, elongated carrier comprising functional groups serving for anchoring the first reaction component of the process sequence,
and
- an arrangement of successive reaction and processing zones of a sequence identical with that of the reaction and processing steps of the process sequence of the respective synthesis, and comprising means for successively contacting the carrier with the liquids of the reaction and processing zones.

In accordance with preferred embodiments, the apparatus comprises an open- or closed-loop carrier the lenght of which is at least such that it corresponds to the total residence time necessary for all reaction and processing steps of the process sequence for a given moving speed, and the carrier path length of the individual reaction and processing steps corresponds to the residence time necessary for the respective reaction or processing steps for the given moving speed. The means for contacting the carrier with the reactant and processing liquids are preferably rolls or rollers defining a required path lenght in the respective liquid vessels.

The solid carrier is advantageously in the form of a band. The carrier band is preferably led, e.g. by means of a series of rollers, through the appropriate reagents and washing solvents so that the individual reactants, e.g. amino acids, are stepwise bonded , and in the last step the synthesized polymer, e.g. peptide, is cleaved off. The band with the recovered original functional groups can be returned to the beginning of the whole sequence of operations, and the whole cycle may be repeated again, if suitable carriers and spacers are used. Unlike in the classical arrangement of solid phase synthesis, in which the individual steps have to be performed subsequently with the whole carrier, in the method according to this invention all the synthetic steps proceed simultaneously, at locally different sites. Another advantage is that the product is obtained in a continuous process instead of a single batch process.

The method may be used for the most important strategies of solid phase peptide synthesis, i.e. the so-called Boc and FMOC strategies. Nevertheless, the latter method FMOC, using the base-cleavable N-amino-protecting group, allows a substantially easier arrangement: less washing steps are required , and already known spacers, enabling acid-catalyzed liberation of the final peptide may be employed. Depending on the character of the carrier, the use of other protecting groups is also possible. Thus, e.g. in such a synthesis using a relatively polar cotton band as the carrier, protection was achieved with the o-nitrobenzenesulfenyl group for which a nonpolar carrier of polystyrene type is not suitable. The synthesis can be monitored visually by an acid-base indicator (CS patent application PV 209-88).

The method and apparatus according to the invention are applicable especially for manufacturing purposes, the desired final product, such as a polypeptide or polynucleotide, being produced continuously in amounts corresponding to the capacity and size of the carrier used.

The continuous solid phase synthesis may also be adjusted for small-scale preparation of many biopolymers such as polypeptides and polynucleotides: in such cases the carrier is preferably a thread which is successively introduced into solutions of the corresponding activated amino acids, and when the synthesis has ended the whole carrier is subjected to the isolation procedure. In this way, a whole series of peptides of various lenght may be prepared, their number being limited by the capacity of the individual segments.

In many cases the desired end product is a macro-molecule which is directly bonded to the polymeric carrier. If the form of this carrier is suitable, the synthesis may be performed directly on the carrier which has been only processed mechanically or chemically.

The (condensation) reactions may be accelerated either by working in an ultrasonic bath or by leading the carrier band over a heated roller. Washing of the carrier is facilitated by pressing between two rollers and passing through, or moistening with the corresponding solvent.

Suitable carriers are polymeric materials containing suitable functional groups, such as cellulose (e.g. paper or cotton bands), modified polystyrene, polytetrafluoroethylene (teflon), polyamides, modified polypropylene, functionalized glass, etc.

Examples for suitable carriers which have been tested experimentally are cotton tissues, glass fibres modified by aminopropyl groups, or polypropylene membranes modified by hydroxypropylacrylate (product of MilliGen). Peptides obtained on all these carriers were analyzed thoroughly and were found identical. However, cotton is superior because it has a most favourable combination of good mechanical and physicochemical properties.

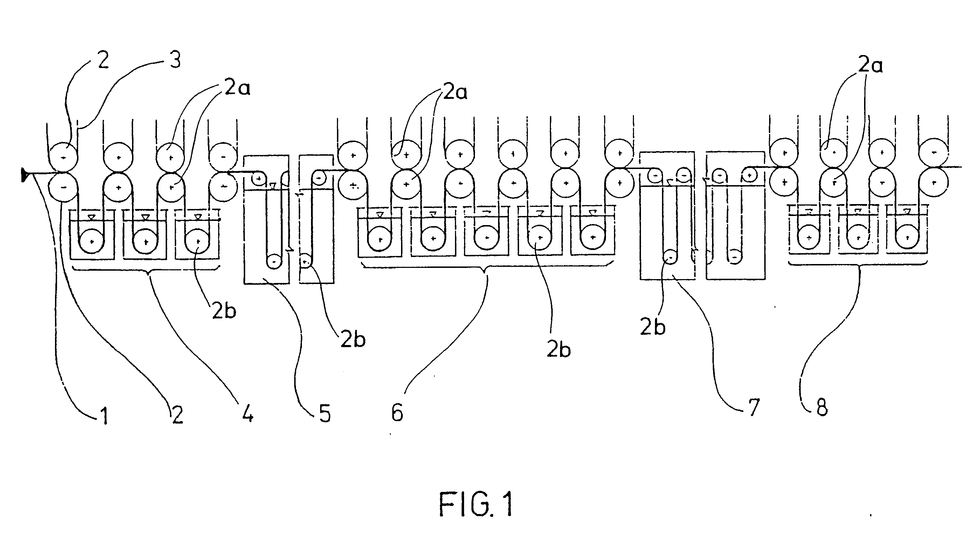

An example of the arrangement of one synthesizer segment of the apparatus according to the present invention is shown in Figure 1. The carrier 1 coming e.g. from the preceding synthetic step is pressed between a pair of rollers 2 which removes most of the liquid brought from the preceding step. The removal is facilitated by simultaneous introduction of another porous material 3, such as paper or textile material, between the rollers. The carrier advances into the washing bath 4 which comprises 3 stages and represents a processing zone, and is pressed again between rollers 2a. This operation is repeated several times, and then the carrier 1 is introduced into a solution of reagent removing the N-amino-protecting group in a reaction zone 5. The duration of the contact of the carrier with this solution is determined by the lenght of the pathway through the bath. This is achieved by using a system of pulleys 2b which are also provided in the processing zones 4, 6 and 8, and in the reaction zones 5 and 7. From the bath of zone 5 the carrier proceeds further into a series of washing baths, representing a processing zone 6, and then into a vessel containing the activated protected amino acid (e.g. the symmetrical anhydride) in the reaction zone 7. Here the path length is again adjusted by means of the pulleys 2b so as to achieve the residence time required for completion of the condensation reaction. The carrier 1 then enters again the system of rollers 2a and washing baths in the processing zone 8, and after washing it can undergo the next cycle in which another amino acid is attached and which is performed in a segment analogous to the above-mentioned one. All the baths must be continuously regenerated so as to keep a suitable concentration of reagents and avoid accumulation of contaminants.

In principle, the same equipment may be used e.g. for the cleavage of protecting groups and washing, and the carrier is introduced into separated vessels only for the condensation reaction. This applies particularly to the above-mentioned embodiment with a thread-like carrier.

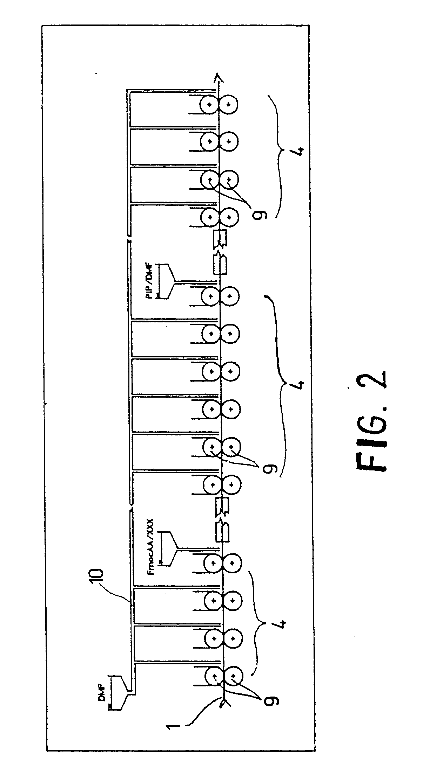

A more simple arrangement of a synthesizer segment of an apparatus according to this invention is shown in Figure 2. In this case, the carrier 1 is moistened with the solvent or a solution containing the activated component (e.g. FMOC-amino acid in a solvent, or another reagent solution (piperidine/DMF)). Because of the high soaking capacity of the carriers used, the volume of soaked liquid is sufficient for successful execution of the corresponding reaction (e.g. mass of dimethylformamide per mass of the fabric: glass 0.4 g/g, polypropylene 1.0 g/g, cotton 1.1 g/g). The carrier 1 is guided and pressed between pairs of rollers 9; the washing zones 4 are again provided after the reaction zones. The solvent (DMF) is supplied by means of a link system 10.

This version is more economical concerning the solvent consumption, and, moreover, it guarantees high quality of the solvents because fresh solvent is continuously supplied.

For practical purposes it should be realized that even with relatively small dimensions of the carrier (e.g. width 3 cm), low substitution (3 $\mu$mol/cm) and low band velocity (5 cm/min, i.e. 3m/h) the equipment is capable of producing e.g. 65 g of a peptide of a molecular mass of 1000 during 24 h. This fact allows the use of carriers with extremely low substitution (e.g. modified glass fabrics).

The method and apparatus according to the present invention are illustrated by the following examples.

## Example 1

A cotton band (width 3 cm, length 280 cm, specific mass 23,1 mg/cm) was shaken with a mixture of trifluoroacetic acid (25 ml) and dichloromethane (75 ml) for 15 min, washed successively with dichloromethane (3 x 100 ml), a 10 % solution of diisopropylethylamine in dichloromethane (2 x 100 ml) and dichloromethane (3 x 100 ml), and dried in vacuo. The band was then shaken with a mixture of t-butyloxycarbonylglycine (4.4 g), dicyclohexylcarbodiimide (5.15 g), dimethylaminopyridine (3.05 g), and dimethylformamide (100 ml) for 4 h at room temperature. After washing with dimethylformamide (3 x 100 ml), ethanol (3 x 100 ml) and dichloromethane (3 x 100 ml) and drying, the capacity of the carrier was 3.1

μmol/cm.

Example 2

Estimation of the carrier capacity.

A sample of the carrier (1 cm) was shaken with a 25 % solution of trifluoroacetic acid in dichloromethane for 15 min and washed successively with dichloromethane (3 x), a 10 % solution of diisopropylethylamine in dichloromethane, and with dichloromethane. It was then shaken with fluorenylmethyloxycarbonylglycine, dicyclohexylcarbodiimide and N-hydroxybenzotriazole in the presence of a small amount of bromophenol blue until the blue colour of the carrier disappeared. The sample was then washed with dimethyformamide (3 x) and shaken with a 20 % solution of piperidine in dimethylformamide for 15 min. The piperidine solution was separated, and made up to 50 ml with the dimethylformamide employed for the above washing of the sample; then the extinction of the solution at 301 nm was read. The carrier capacity was then calculated from the extinction value and the known extinction coefficient of the adduct of fulvene with piperidine (7 800).

Example 3

Synthesis of des-glycyl-methionine-enkephalin.

A cotton band (20 cm) with bound t-butyloxycarbonylglycine was shaken with a 25 % solution of trifluoroacetic acid in dichloromethane for 15 min and washed successively with dichloromethane (3 x), a 10 % solution of diisopropylamine in dichloromethane (2 x), and dichloromethane (3 x). It was then shaken overnight with 2,4,5-trichlorophenyl-3-(4-fluorenylmethyl-oxycarbonylmethionyl-oxymethylphenoxy)-propionate (3 mmol) and N-hydroxybenzotriazole (3 mmol) in dimethylformamide (15 ml) at room temperature. Then the carrier substitution was determined by removal of the FMOC group (1 μmol/cm), and the carrier was acetylated with a mixture of acetic anhydride (5 ml) and pyridine (10 ml) for 30 min at room temperature. The carrier was washed with dimethylformamide (3 x) and dichloromethane (3 x). The cotton band was subjected to three cycles of solid phase peptide synthesis, described in Example 5 using fluorenylmethyloxycarbonylphenylalanine, fluorenylmethyl oxycarbonylglycine and t-butyloxycarbonyl-0-t-butyltyrosine as protected amino acids. The carrier was then introduced into a solution consisting of 50 % of trifluoroacetic acid, 5 % of dimethylsulfide and 45 % of dichloromethane. After 2 h the band was washed with the same solution, and the combined eluates were concentrated in vacuo to a small volume. The product was precipitated with absolute ether, separated, dried, and dissolved in 1 M acetic acid. The solution was then filtered and freeze-dried to give 30 mg of the crude product which was purified by high pressure liquid chromatography on a column (Vydac C18, 250 x 10 mm). The chromatography furnished 8.6 mg of the product which was pure according to high pressure liquid chromatography (column Spherisorb ODS II 250 x 4 mm, k = 6.4; 75 % methanol in 0.05 % trifluoroacetic acid) as well as thin-layer chromatography in four systems, and electrophoresis at two pH values.

Amino acid analysis: Gly 1.00, Met 0.92, Tyr 0.89, Phe 0.96.
Mass spectrometry: 517 (M + H).

| Analysis for $C_{25}H_{32}N_4O_5S \cdot CF_3COOH \cdot 2H_2O$ (666.7): | | | |
|---|---|---|---|
| calculated: | 48.64 % C, | 5.59 % H, | 8.40 % N; |
| found: | 48.87 % C, | 5.32 % H, | 8,56 % N. |

Example 4

Synthesis of methionine-enkephalin.

The synthesis was executed as described in Example 3 except that the condensation of protected glycine was repeated twice.

Yield 15,6 mg of product, pure according to high-pressure liquid chromatography (column of Spherisorb ODS II 250 x 4 mm, k = 5.8, 75 % methanol in 0.05 trifluoroacetic acid), thin-layer chromatography in four solvent systems, and electrophoresis at two pH values.

Amino acid analysis: Gly 2.00, Met 0.90, Tyr 0.86, Phe 0.99.
Mass spectrometry: 574 (M + H).

| Analysis for $C_{27}H_{35}N_5O_7S \cdot CF_3COOH \cdot 2H_2O$ (723.7): | | | |
|---|---|---|---|
| calculated: | 48.13 % C, | 5.57 % H, | 9.68 % N; |
| found: | 48.58 % C, | 5.38 % H, | 9.42 % N. |

Example 5

Cycle employed for attachment of an amino acid residue using fluorenylmethyloxycarbonyl protecting group.

The carrier is successively introduced into the following solutions and solvents:
1. ethanol
2. dichloromethane
3. dichloromethane
4. dichloromethane
5. dimethylformamide
6. dimethylformamide
7. dichloromethane
8. dichloromethane
9. fluorenylmethyloxycarbonylamino acid, dicyclohexylcarbodiimide, N-hydroxybenzotriazole - all 0.2 M solutions in dimethylformamide
10. dimethylformamide
11. dimethylformamide
12. ethanol.

After passage through each bath the carrier is introduced between rotating rollers, together with a strip of filtering paper which absorbs the solvent which has been pressed out. The carrier is then introduced into the next bath. The immersion time in the individual baths is 2 min except for bath No. 4 in which the carrier is immersed for 20 min, and bath No. 9 in which the time depends on the disappearance of the blue colour of the indicator (bromophenol blue).

Example 6

Cycle employed for attachment of an amino acid residue using the t-butyloxycarbonyl protecting group.

The carrier is successively introduced into the following solutions and solvents:
1. ethanol
2. dichloromethane
3. dichloromethane
4. 45 % trifluoroacetic acid and 5 % anisol in dichloromethane

7

5. dichloromethane

6. dichloromethane

7. dichloromethane

8. 10 % diisopropylamine in dichloromethane

9. dichloromethane

10. dichloromethane

11. dimethylformamide

12. dimethylformamide

13. t-butyloxycarbonylamino acid, dicyclohexylcarbodiimide, N-hydroxybenzotriazole - all 0.2 M solutions in dimethylformamide

14. dimethylformamide

15. dimethylformamide

16. ethanol.

After passage through each bath, the carrier passes between rotating rollers together with a strip of filtrating paper which absorbs the solvent which has been pressed out. The carrier is then introduced into the next bath. The immersion time in the individual baths is 2 min except for bath No. 4 in which the carrier is immersed for 20 min, bath No. 8 (6 min), and bath No. 13 in which the time depends on the disappearance of the blue colour of the indicator (bromophenol blue).

Example 7

Synthesis of bound methionine-enkephalin.

A band that had been processed as described in Example 1 was successively acylated with fluorenylmethyloxycarbonyl-protected methionine, phenylalanine, glycine, glycine and o-nitrophenylsulfenyltyrosine, using the procedure described in Example 5. The o-nitrophenylsulfenyl protecting group was removed by treatment with 7.5 M hydrogen chloride in methanol (20 min).

The enkephalin content as determined by amino acid analy-sis was 1.6 $\mu$mol/cm of the carrier.

Example 8

Synthesis of methionine-enkephalin.

A piece of a polypropylene membrane modified with hydroxypropylacrylate (3 x 3 cm) was acylated with FMOC-Gly in the same manner as described in Example 1. By cleavage of the FMOC groups the substitution was found to be 0.1 mmol/g.

The synthesis of the peptide was performed as described in Example 4. After the same treatment, a peptide was ob tained which could not be distinguished from that prepared in Example 4.

Example 9

Synthesis of methionine-enkephalin.

A piece of fibre glass modified with aminopropyl groups (25 x 6 cm, 1 g, 1.7 $\mu$mol) was acylated overnight with 2,4,5-trichlorophenyl-3-(4-fluorenylmethyl-oxycarbonylmethionyl-oxymethylphenoxy)-propionate (0.05 mol) and N-hydroxybenzotriazole (0.05 mmol) in dimethylformamide (0.4 ml) at room temperature. The carrier was then washed with dimethylformamide (3 x) and subjected to three cycles of solid phase peptide synthesis, described in Example 5 using fluorenylmethyloxycarbonylphenylalanine, fluorenylmethyloxycarbonylglycine and t-butyloxycarbonyl-0-t-butyltyrosine as protected amino acids. The carrier was then introduced into a solution consisting of 95 % of trifluoroacetic acid and 5 % of dimethyl sulfide. After 2 h the band was washed with the same solution, and the combined eluates were evaporated in vacuo, dissolved in 3 M acetic acid and lyophilized. The product was dissolved in 1 M acetic acid, and the solution was applied on a column (Vydac C18 250 x 4 mm). The chromatography furnished 0.3 mg of the product which was pure according to high pressure liquid chromatography and identical with the above-described methionine-enkephalin.

Example 10

Synthesis of model peptides.

A cotton band that had been acylated with t-butyloxycarbonylalanine as described in Example 1 was subjected to the synthesis cycles described in Example 6, and successive attachment of t-butyloxycarbonyl-protected amino acids was performed. The obtained modified carrier was then subjected to either alkaline hydrolysis (0.1 M-NaOH, 2 h at room temperature) or ammonolysis (saturated ammonia solution in methanol, 4 h at room temperature).

The following peptides were obtained by this procedure:

H-Pro-Leu-Gly-Ala-OH

H-Pro-Leu-Gly-Ala-NH$_2$

H-Leu-Pro-Gly-Ala-OH

H-Leu-Pro-Gly-Ala-NH$_2$.

However the described method of removing the peptide from the carrier is accompanied by formation of several side products due to the glycine in position 3.

## Claims

1. A method for the continuous synthesis of oligomeric and polymeric organic compounds, particularly of biopolymers and preferably of polypeptides and polynucleotides, characterized in that
- a solid, insoluble, elongated carrier is used comprising functional groups serving for anchoring the first reaction component of the process sequence of the respective synthesis either directly or by means of a spacer,
and
- the individual reaction and processing steps are carried out in successive zones of a sequence identical with that of the reaction and processing steps of the process sequence moving in one direction given by the longest dimension of the carrier.

2. The method according to claim 1, characterized in that all reaction and processing steps of the process sequence are carried out at the same time in different parts of the carrier.

3. The method according to claims 1 and 2, characterized in that the carrier is successively subjected to the reaction and processing steps of the process sequence.

4. The method according to claims 1 to 3, characterized in that the carrier is moved through the successive zones, preferably with a predetermined, constant speed.

5. The method according to claims 1 to 4, characterized in that a planar, band-shaped, or thread-like carrier is used.

6. The method according to claims 1 to 5, characterized in that a carrier is used made of a cellulose material, such as cotton fabric or paper, modified polystyrene, polytetrafluoroethylene, polyamide, modified polypropylene, or functionalized glass, such as modified glass fibers or modified fibre glass.

7. The method according to claims 1 to 6, characterized in that an open- or closed-loop carrier is used the length of which at least corresponds to the total residence time necessary for all reaction and processing steps of the process sequence for a given moving speed.

8. The method according to claims 1 to 7, characterized in that the synthesis process is stopped at a stage where a series of intermediate compounds and eventually also the derived final product corresponding to the respective series of reaction steps of the process sequence have been formed in the corresponding parts of the carrier, and these compounds are removed from the carrier either continuously or in a batch-like manner from the respective parts of the carrier.

9. The method according to claims 1 to 8, characterized in that the final product is removed from the carrier after completion of the total process sequence in all parts of the carrier, preferably in a batch-like manner.

10. An apparatus for the continuous synthesis of oligomeric and polymeric organic compounds, particularly for carrying out the method according to claims 1 to 9, comprising

- a solid, insoluble, elongated carrier (1) comprising functional groups serving for anchoring the first reaction component of the process sequence,

and

- an arrangement of successive reaction and processing zones (4, 5, 6, 7, 8) of a sequence identical with that of the reaction and processing steps of the process sequence of the respective synthesis, and comprising means (2a, 2b) for successively contacting the carrier (1) with the liquids of the reaction and processing zones (4, 5, 6, 7, 8).

11. The apparatus according to claim 10, comprising means for continuously moving the carrier (1) through the reaction and processing zones (4, 5, 6, 7, 8) with a predetermined, constant speed.

12. The apparatus according to claims 10 and 11, characterized in that it comprises an open- or closed-loop carrier (1) the length of which at least corresponds to the total residence time necessary for all reaction and processing steps of the process sequence for a given moving speed,

and the carrier path length of the individual reaction and processing steps corresponds to the residence time necessary for the respective reaction or processing step for the given moving speed.

13. The apparatus according to claims 10 to 12, characterized in that the carrier (1) is planar, band-shaped or thread-like.

14. Carrier for use in the method according to claims 1 to 9 and/or in the apparatus according to claims 10 to 13, characterized in that it is made of a cellulose material, such as cotton fabric or paper, modified polystyrene, polytetrafluoroethylene, polyamide, modified polypropylene, or functionalized glass, such as modified glass fibers or modified fibre glass.

15. Use of the apparatus according to claims 10 to 13 and/or of the carrier according to claim 14 for and/or application of the method according to claims 1 to 9 to peptide or polynucleotide synthesis.

FIG. 1

EP 0 385 433 A2

FIG. 2

EP 0 385 433 A2